Europäisches Patentamt

European Patent Office

Office européen des brevets

(19)

(11) Veröffentlichungsnummer: **0 124 896**
**B1**

# EUROPÄISCHE PATENTSCHRIFT

(12)

(45) Veröffentlichungstag der Patentschrift:
31.01.90

(21) Anmeldenummer: 84105066.9

(22) Anmeldetag: 04.05.84

(51) Int. Cl.⁴: **C 12 N 7/02,** G 01 N 33/00,
**C 12 Q 1/70,** A 61 K 35/16

(54) Verfahren zur Gewinnung von Viren bzw. Virusantigenen und deren Anwendung in Diagnose und Therapie (Impfstoff).

(30) Priorität: 05.05.83 DE 3316464

(43) Veröffentlichungstag der Anmeldung:
14.11.84 Patentblatt 84/46

(45) Bekanntmachung des Hinweises auf die Patenterteilung:
31.01.90 Patentblatt 90/5

(84) Benannte Vertragsstaaten:
AT BE CH DE FR GB IT LI LU NL SE

(56) Entgegenhaltungen:
EP-A- 0 058 993
WO-A-80/02598
WO-A-82/03330
US-A- 4 210 580

INFECTION AND IMMUNITY, Band 40, Nr. 3, Juni 1983,
Seiten 876-881; I. JULKUNEN et al.: "Interaction of viral
envelope glycoproteins with fibronectin"

(73) Patentinhaber: Seelig, Renate, Dr., Kriegstrasse 99,
D-7500 Karlsruhe (DE)
Patentinhaber: Seelig, Hans Peter, Prof. Dr.,
Kriegstrasse 99, D-7500 Karlsruhe (DE)
Patentinhaber: Liehr, Heinrich, Prof. Dr., An der
Steinkaul 14, D-6601 Saarbrücken-Bübingen (DE)
Patentinhaber: Pott, Gerhard, Dr., Dorbaumstrasse 14,
D-4400 Münster (DE)

(72) Erfinder: Seelig, Renate, Dr., Kriegstrasse 99,
D-7500 Karlsruhe (DE)
Erfinder: Seelig, Hans Peter, Prof. Dr., Kriegstrasse 99,
D-7500 Karlsruhe (DE)
Erfinder: Liehr, Heinrich, Prof. Dr., An der Steinkaul 14,
D-6601 Saarbrücken-Bübingen (DE)
Erfinder: Pott, Gerhard, Dr., Dorbaumstrasse 14,
D-4400 Münster (DE)

(74) Vertreter: Patentanwälte Müller-Boré, Deufel, Schön,
Hertel, Lewald, Otto, Postfach 26 02 47 Isartorplatz 6,
D-8000 München 26 (DE)

## Beschreibung

Die diagnostischen Schwierigkeiten beim Nachweis einer Virämie, d.h. der Nachweis freier Viren im zirkulierenden Blut sind bekannt. Mit Ausnahme weniger Virusinfektionen, wie z.B. der Hepatitis B, gelingt der Nachweis freier Viren im Blut in der Regel nicht.

Viele Erregerviren von Viruskrankheiten sind bekannt und Diagnose und zum Teil Behandlung sind über die gebildeten Antikörper möglich. Dagegen besteht immer noch Unklarheit, ob bei der sogenannten Non A-Non B-Hepatitis überhaupt ein Virus als Erreger anzunehmen ist, da ein solches Virus bisher nie zweifelsfrei identifiziert werden konnte. Demgemäß war es auch bis jetzt nicht möglich, gezielt Antikörper für die Diagnostik oder gar Impfserum zu gewinnen.

Non A-Non B-Hepatitiserkrankungen sind in der Literatur ausführlich und zahlreich beschrieben. Siehe z.B. Geretry R.J. Non A-Non B-Hepatitis, Academic Press, New York, 1981.

Ausgehend von der Feststellung, daß z.B. die Non A-Non B-Hepatitis hauptsächlich durch Blutderivate mit hohem Fibronectingehalt übertragen wird, wurde gefunden, daß Virusantigene bzw. Viren an Fibronectin gebunden und dadurch maskiert werden, was eine Erklärung für die diagnostischen Schwierigkeiten, freie Antigene bzw. Viren im Blut nachzuweisen, bietet.

Es wurde weiterhin gefunden, daß die Anreicherung oder Isolierung von Fibronectin und seinen Spaltprodukten aus dem zirkulierenden Blut und aus Körperflüssigkeiten zur Gewinnung von Fibronectin-gebundenen Antigenen bzw. Viren und damit zur Gewinnung solcher Antigene bzw. Viren und zur Diagnostik der Viruserkrankung eingesetzt werden kann.

Fibronectin ist ein Glycoprotein mit einem Molekulargewicht von 440 000 Dalton. Es wird in fast allen Körperzellen produziert und insbesondere von Endothelzellen und anderen Hohlräumen auskleidenden Zellen, sowie von Zellen des Bindegewebssystem an den Zelloberflächen abgelagert. Das Zelloberflächenglycoprotein unterscheidet sich geringfügig von dem Fibronectin, das im Blut des Menschen zu finden ist. Synonyma für Fibronectin sind: kälteunlösliches Globulin, opsonierendes Glycoprotein, Zelladhäsionsfaktor, Zelloberflächenprotein, Antigelatine-Faktor.

Es ist heute bekannt, daß Fibronectin als Ankerprotein für Kollagene und weitere Bestandteile des Bindegewebes dient. Das Ausmaß der Konzentration an der Zelloberfläche spielt eine Rolle für die Ausbreitung von Tumorzellen. Für klinischpraktische Belange wichtig, hat sich gezeigt, daß Fibronectin im Blut als ein Hauptprotein der Opsonine die Phagozytose durch die Zellen des retikuloendothelialen Systems von Leber, Milz, Lunge und Knochenmark unterstützt. Die Bindung von Einzelstrang- und Doppelstrang-DNS, Heparin, Kollagen, Fibrin und dessen Spaltprodukten, Proteoglykanen, Zellen und Zelldetritus, sowie Staphylokokken an Fibronectin wurde beschrieben:

H.G. Klingenmann: Fibronectin

Die gelben Hefte (Behring XXII, 1982), S. 154–161

G. Pott, P. Zündorf, U. Gerlach, B. Voss, J. Rautenberg: Die Bedeutung struktureller Glykoproteine, dargestellt am Beispiel der Fibronectine — biochemische Grundlagen und klinische Auswertung, Z. Gastroenterologie 20 (1982) S. 649–658.

Die Bestimmung von Fibronectin im Blut zur Diagnostik der verminderten Funktion des retikuloendothelialen Systems und der therapeutische Einsatz von Fibronectin bei Sepsis und verzögerter Wundheilung finden zur Zeit Eingang in die Therapie.

Bei Untersuchungen über die Fähigkeit von Fibronectin, andere Substanzen zu binden, hat sich, wie erwähnt, überraschenderweise gezeigt, daß Fibronectin an bestimmten Stellen des Moleküls auch verschiedene Virusantigene bzw. Viren bindet, z.B. Antigene der Hepatitisviren A und B. Durch Spaltung des Proteins in Bruchstücke und Analyse über Hochdruck-Flüssigkeitschromatographie konnten solche Peptide, nämlich Kollagen nicht bindende Bruchstücke des Fibronectins mit erhöhter Bindungsfähigkeit für verschiedene Antigene bzw. Viren, gefunden werden.

Gereinigtes Fibronectin und seine Bruchstücke wurden mit gegebenenfalls radioaktiv markierter Virussuspension inkubiert und in der Kälte mittels Zentrifugation sedimentiert. Überstand und Sediment wurden auf Radioaktivität oder, beim Einsatz nicht markierter Viren die einzelnen Fraktionen mittels radioimmunologischer Nachweismethoden auf die Anwesenheit der eingesetzten Viren untersucht. Die Verdrängungsreaktion der an Fibronectin gebundenen Viren von ihrer Bindungsstelle war mit anderen Viren, z.B. Hepatitisvirus A, möglich.

Diese Versuche zeigten, daß verschiedene Viren, insbesondere Hepatitisviren, an Fibronectin bzw. an Spaltprodukte von Fibronectin gebunden werden. Gleichzeitig ergab sich, daß ein Teil der Viren durch die Fibronectinbindung derart maskiert wurde, daß sie immunologisch nicht mehr (z.B. durch die übliche Antigen-Antikörper-Reaktionen) nachweisbar waren.

Die Freisetzung und Gewinnung von derart maskierten Antigenen bzw. Viren, insbesondere des Non A-Non B-Hepatitisvirus oder jedenfalls des Antigens, bietet die Möglichkeit der Isolierung der Antigene bzw. Viren, der gezielten Züchtung von Viren und Antikörpern, damit der Gewinnung von Impfstoff einerseits und der Anwendung in der Diagnostik andererseits.

Die Gewinnung und Freisetzung des Virus erfolgt aus Fibronectin von Virusträgern. Diese Methode kann aber nicht nur dazu benutzt werden, an Fibronectin gebundene Viren zu gewinnen, sondern Fibronectin das nicht von Viren besetzt ist kann auch benutzt werden, um zum Beispiel aus dem Stuhl von Virusträgern Viren zu binden und somit abzutrennen und dann zu gewinnen. Dies ist mit dem Prinzip der Affinitätschromatographie zu vergleichen. Die Zerstörung des Bindungsmechanismus erfolgt in an sich bekannter Weise, insbesondere durch spezielle Enzyme und/oder Detergentien oder durch Verdrängungsreaktionen oder Erhitzen. Als Beispiel für solche an sich bekannte Maßnahmen sei auf Zerstörung der Antigen/Antikörperbindung (z.B. durch pH-Erniedrigung) oder einer Substrat/Rezeptorbindung verwiesen.

Die grundsätzlichen Verfahrensschritte sind wie folgt:

1a) Anreicherung von Fibronectin aus Körperflüssigkeit, insbesondere Blutplasma, durch Kryopräzipitation, z.B. Zentrifugation von Plasma bei 0–4°C

1b) Reindarstellung von Fibronectin aus dem dabei erhaltenen Sediment

Das Reinigungsverfahren und die Anreicherung von Fibronectin durch Kryopräzipitation sind bekannt.

1c) Gegebenenfalls Herstellung von Spaltprodukten aus Fibronectin (durch Proteasebehandlung, z.B. mit Subtilisin) und Ermittlung derjenigen Spaltprodukte, die Bindungsstellen für das gewünschte Virus enthalten.

Wie erwähnt läßt sich das Verfahren auch zur Isolierung von Viren aus biologischem Material, wie z.B. aus Stuhl anwenden, wobei dann folgende grundsätzliche Verfahrensschritte angewandt werden:

A) Herstellung einer Kochsalzaufschwemmung von Stuhl bzw. eines Gewebehomogenats und Abzentrifugieren der groben Bestandteile (8000 g),

B) Zugabe von Fibronectin bzw. eines geeigneten Fibronectinbruchstückes zum Überstand und Bindung von im Überstand befindlichen Viren und/oder Virusantigenen,

C) Abtrennung des Fibronectins bzw. der Fibronectinbruchstücke zusammen mit den hieran gebundenen Viren und/oder Virusantigenen.

Dieses Fibronectin bzw. die Bruchstücke werden wie unter 1a) und 1b) erwähnt angereichert und gereinigt. Die weitere Aufarbeitung erfolgt wie unter 2 bis 5 angeführt.

Die Gewinnung der Viren aus Fibronectin bzw. dessen Bruchstücken gestaltet sich dann wie folgt.

2a) Zerstörung der Bindung zwischen Fibronectin bzw. Fibronectinbruchstück und Virus durch Zugabe spezieller Enzyme und/oder Detergentien insbesondere rezeptorzerstörender Enzyme wie Neuraminidase oder Chymotrypsin oder gegebenenfalls durch Erhitzen.

2b) Alternativ zu 2a) Verdrängung der gebundenen Viren mittels anderer Viren, z.B. Hepatitis A-Virus und/oder Substanzen, die um dieselbe Fibronectinbindungsstelle konkurrieren.

Daran kann sich eine weitere Virusisolierung durch Entfernung der bei den Schritten 2a) oder 2b) verwendeten Hilfsstoffe mittels Adsorptionsverfahren oder mittels Trennverfahren nach Dichte, Molekulargewicht und Ladung der verschiedenen Substanzen und Viren anschließen, wie dies an sich bekannt ist.

Aus der in den Verfahrensschritten 2a) und 2b) erhaltenen Virussuspension oder der gegebenenfalls gereinigten Suspension läßt sich das Virus nachweisen, und zwar

3a) bevorzugt durch Virusantigen-Antikörper-Bindungsreaktion oder

3b) alternativ durch Hämagglutinationsreaktionen.

Das gereinigte Virus und/oder die Virusantigene können zum Nachweis von Antikörpern eingesetzt werden, also z.B. zur Diagnose einer Virusinfektion.

4.1.a) Bevorzugt mittels Antigen-Antikörper-Bindungsreaktionen,

4.1.b) alternativ dazu mittels Neutralisationsverfahren.

Der Nachweis von Antikörper gegen Virusantigene erfolgt

5.1.a) bevorzugt durch Virusantigen-Antikörper-Bindungsreaktionen oder

5.1.b) alternativ dazu durch Neutralisationsverfahren, d.h. die Aufhebung der Agglutination antigenbeladener Partikel oder eines cytopathogenen Effektes durch spezifische Antikörper.

Das Virus und/oder die Virusantigene können dann

5.2.a) zur Erzeugung von Antikörpern mittels aktiver Immunisierung von Laboratoriumstieren, eingesetzt werden, also zur Gewinnung spezifischer Antikörper für diagnostische Zwecke.

5.2.b) Weiterhin ist der Einsatz des abgetöteten Virus und/oder der Virusantigene zur Erzeugung spezifischer Antikörper beim Menschen zur Impfung möglich. Die Freisetzung und die Gewinnung des Virus ermöglicht folglich auch die Herstellung einer Vaczine.

5.3 Die Spaltung der Fibronectin-Virusbindung ermöglicht ein Diagnosekit zum Nachweis von maskierten Viren.

Ein zum Nachweis von an Fibronectin gebundenen maskierten Viren geeigneter Diagnosekit enthält zum Unterschied zu üblichen Diagnosekits die (je nach Aufbau des Diagnosekits) prinzipiell nur markierte oder nicht markierte Antigene sowie markierte oder nicht markierte Antikörper gegen diese Antigene (Viren) enthalten, zusätzliche Reagentien, welche die Freisetzung der gebundenen Viren aus Fibronectin ermöglichen, so daß sie erfaßt werden können. Die Gewinnung von Antigen durch Virusisolierung gemäß den oben beschriebenen Verfahrensschritten und nachfolgende Viruszüchtung folgen dann in an sich für die Vaczineherstellung bekannter Weise.

Wie erwähnt ist das Reinigungsverfahren und die Anreicherung von Fibronectin durch Kryopräzipitation bekannt.

Nicht bekannt war die Bindung von Viren bzw. Antigene an Fibronectin durch Virusbindungsstellen im Fibronectinmolekül und seinen Spaltprodukten, die Maskierung von Viren bzw. Antigenen durch Bindung an Fibronectin und an Spaltprodukte von Fibronectin, so daß sie nicht mehr immunologisch nachweisbar sind sowie die Möglichkeit, gebundene Viren aus der Bindung freizusetzen und somit der Einsatz des bekannten Reinigungsverfahrens und der Anreicherung von Fibronectin durch Kryopräzipitation in Kombination mit einer Verdrängungsreaktion oder einer enzymatischen oder thermischen Abspaltung zur Isolierung von Viren bzw. Virusantigenen,

die Möglichkeit zur Isolierung von Viren bzw. Virusantigenen aus virusantigenhaltigen Suspensionen durch Zugabe von Fibronectin bzw. Fibronectinspaltprodukten und insbesondere,

da nicht bekannt war, daß Non A-Non B-Hepatitis-Virus durch Bindung an Fibronectin und seine Spaltprodukte maskiert wird, die Gewinnung von Non A-Non B-Hepatitis-Virus-Suspensionen, also des bis jetzt nicht bekannten Virus und somit des Antikörpers.

Die immunologischen Testverfahren und Diagnosekits sind grundsätzlich bekannt. Ein Testverfahren zur Diagnose von Non A-Non B-Hepatitis war bisher nicht bekannt, da der dazu erforderliche Virus (= Antigen und somit auch der Antikörper) fehlte. Diese Diagnoseverfahren und ein Testkit dazu sind nun möglich.

Die folgenden Beispiele erläutern die Erfindung.

Beispiel 1
Übersicht über den Verfahrensablauf

Es sind die einzelnen Schritte am Beispiel der Gewinnung von Fibronectin und Virus bzw. Antigen aus Blut aufeinanderfolgend dargestellt:

1. Blut wird mit 0,11 molarem Natrium-Citrat im Verhältnis 10 : 1 (Volumen zu Volumen) versetzt.

2. Das Plasma wird durch Zentrifugation (1000 g) abgetrennt.

3. Es folgt die Kryopräzipitation von Fibronectin aus Blutplasma durch Zentrifugation mit 2000 g bei 0°C.

3a. Der Überstand wird dekantiert.

3b. Das Sediment enthält angereichertes Fibronectin. Es wird bei Zimmertemperatur stehen gelassen. Dabei geht das ausgefallene Kryopräzipitat wieder in Lösung.

4. Es folgt die Reinigung von Fibronectin und die Herstellung von Fibronectinspaltprodukten, falls dies gewünscht ist.

4a. Das Kryopräzipitat wird auf einer das 20-fache Ausgangsvolumen des Plasma enthaltenden Säule Ampholine® (pH-Gradient 3 bis 9) aufgetragen und 90 Minuten bei 1500 V, 30 W elektrofokussiert. Die bei pH 4,9 konzentrierten Proteine werden gesammelt und 48 Stunden gegen 0,1 M NaCl dialysiert. Die Konzentration des Fibronectins in diesen Fraktionen wird mit bekannten Methoden (Lasernephelometrie, Mancini-Technik) bestimmt.

4b. Die im Punkt 4.a) erhaltene vorgereinigte Fibronectinfraktion wird auf eine, das 4-fache Ausgangsvolumen an Plasma enthaltende Säule aufgetragen, die mit der Bromcyan-Methode gekoppelte Typ I Kollagen-Sepharose® enthält. Diese Sepharose®-Säule wurde zuvor mit 0,05 M Tris-HCl, 0,1 M NaCl, 0,4 M Epsilon-Aminocapronsäure, pH 7,6, äquilibriert.

Die Elution der gebundenen Proteine erfolgt mit 1 M Kalium-Bromid, 0,05 M Tris-HCl, 0,025 M Epsilon-Aminocapronsäure, pH 5,3.

Die eluierten Proteine werden mittel Druckfiltration eingeengt, der Fibronectingehalt mittels Lasernephelometrie oder Mancini-Technik gemessen, die Reinheit mittels SDS Polyacrylamid-Elektrophorese nach bekannten Verfahren überprüft. Wie erwähnt, ist diese Arbeitsweise bekannt.

4c. Zur Herstellung von Fibronectin-Spaltprodukten wird Fibronectin mit 1/100 des Gewichtsanteils Subtilisin (Carlsberg Typ VIII) in 0,01 M Tris-Puffer, pH 8,0, in Anwesenheit von 2 ml Trasylol®, 0,02 M CaCl bei 37°C 60 Minuten verdaut. Nach Tieffrieren zur Beendigung der Enzymreaktion wird eine Menge von 20 mg Fibronectin auf der unter 4.b) beschriebenen Kollagen-Sepharose® getrennt, die nicht-kollagenbindenden Bruchstücke aus dem Durchlauf werden nach Konzentrierung durch Druckfiltration auf einer 68 × 15 cm messenden Säule Sephacryl® 300 getrennt. Die Äquilibrierung der Säule erfolgt mit 0,05 M Tris-HCl, pH 7,6. Die fibronectinbruchstückhaltigen Fraktionen werden gesammelt und in üblicher Weise konzentriert. Die Proteinbestimmung erfolgt nach Lowry. Die Bruchstücke werden für die Virusbindungen eingesetzt.

Freisetzung und Gewinnung der gebundenen Viren:

5. Die Lösung, die entweder angereichertes Fibronectin, gereinigtes Fibronectin oder Fibronectin-Spaltprodukte enthält, wird

5.a mit speziellen Enzymen, insbesondere Neuraminidase oder Chymotrypsin und/oder Detergentien, z.B. SDS versetzt oder auf 80°C (10 min) erhitzt und solchermaßen der Bindungsmechanismus zwischen Fibronectin und Virus zerstört.

5.b Alternativ dazu können angereichertes Fibronectin, gereinigtes Fibronectin oder Fibronectin-Spaltprodukte enthaltende Lösungen mit Substanzen oder Viren, z.B. Hepatitis A-Virus, versetzt werden, die um die gleiche Virusbindungsstelle am Fibronectin konkurrieren. Dies führt zu einer kompetitiven Verdrängung und damit Freisetzung von an Fibronectin-Bindungsstellen gebundenen Viren.

6. Gewünschtenfalls erfolgt die weitere Reinigung solcherart aus Fibronectin oder aus Fibronectin-Spaltprodukten freigesetzter Viren und die Entfernung der zur Freisetzung eingesetzten Hilfsstoffe, wie Enzyme, Detergentien oder Viren, mittels Dichtegradienten-Zentrifugation in einem Cäsium-Chlorid-Gradient nach bekannten Verfahren.

7. Die einzelnen Fraktionen des Gradienten werden

a) eluiert,

b) deren Dichte bestimmt und

c) gegen 0,15 molares NaCl dialysiert.

8. Der Nachweis der entsprechend ihrer Dichte in den einzelnen Fraktionen enthaltenen Viren erfolgt mittels Antigen-Antikörper-Bindungsreaktionen.

Die weiteren Beispiele erläutern Grundlagen der Gewinnung und Einzelschritte.

Beispiel 2
Nachweis der Virusbindung an Fibronectin-Antikörperreaktion durch Fibronectin und seine Spaltprodukte

Mit Hepatitis-A-Virus-Antikörpern beschichtete Polystyrol-Kugeln wurden in eine Hepatitis-A-Virus-Suspension eingebracht und die Bindung des Hepatitis-A-Virus in bekannter Weise durch Zugabe von mit $^{125}$J-markiertem Hepatitis-A-Antikörper nachgewiesen. Dieser radioimmunologische Nachweis von Hepatitis-A-Virus gelingt nicht mehr, wenn zur Virussuspension Fibronection oder spezielle, nämlich Kollagen nicht bindende Bruchstücke des Fibronectins zugegeben wurden. Hingegen gelingt der radioimmunologische Nachweis, wenn das Fibronectin bzw. die genannten Bruchstücke nach bzw. vor der Antigen-Antikörper-Bindung mit dem Antikörper in Kontakt gebracht werden. Das Fibronectin bzw. seine Bruchstücke beeinträchtigen also nicht die Bindungsfähigkeit des Antikörpers, sie maskieren jedoch das Antigen, so daß dieses nicht mehr vom Antikörper gebunden werden und demnach auch nicht mehr radioimmunologisch nachgewiesen werden kann.

Es wurde noch ein weiteres Virusantigen – Rötelnantigen – (der Firma Organon) auf Bindung und Maskierung im Hämagglutinations-Hemmtest geprüft. Dieser in der Virologie übliche Hämagglutinations-Hemmtest wird nach bekannten Verfahren durchgeführt. Besonders vorbehandelte Schafserythrozyten werden durch Zugabe von Virusantigen, in diesem Fall Rötelnantigen, agglutiniert. Diese Agglutination kann verhindert werden, indem antikörperhaltige Seren vor Zugabe der Schafserythrozyten mit dem Virusantigen (Rötelnagglutinin) zusammengebracht werden. Die Bindung dieses Antigens an die Antikörper verhindert daraufhin die Agglutinierung der Erythrozyten. Um unspezifische Hemmstoffe zu entfernen, werden die Seren zuerst an Kaolin und Schafserythrozyten adsorbiert. Die Zugabe von gleicherart adsorbiertem Fibronection sowie auch von nicht-adsorbiertem Fibronectin oder dessen Spaltprodukte zu Röteln-Agglutinin oder zu nichtantikörperhaltigen Seren verhindert die rötelnantigenspezifische Agglutination der Indikator-Erythrozyten. Entsprechende Kontrollen, die den Ausschluß von Antikörpern und unspezifischen Inhibitoren gewährten, wuden durchgeführt.

Unspezifischer Nachweis der Hepatitis-A- und Hepatitis-B-Virusantigene mittels ihrer Bindung an Fibronectin

Polystyrolkugeln 6,5 mm im Durchmesser wurden in eine Fibronectinlösung (30 µg Fibronectin/ ml, 0,1 M Carbonalpuffer pH 9,2) eingebracht und 16 Stunden bei Zimmertemperatur inkubiert. Nach ausgiebigem Waschen wurden die fibronectinbeladenen Kugeln mit 200 µl Hepatitis-A-Virus-Suspension inkubiert. Die Hepatitis-A-Virus-Suspension wurde im Radioimmunoassay auf die Anwesenheit von Hepatitis-A-Viren überprüft und zur Reinigung von Hepatitis-A-Viren nach bekannten Verfahren (Siegel, G. und Frösner, G., J. Virology 1978, 26, 40–47) eingesetzt. Nach Inkubation über Nacht bei Zimmertemperatur wurde die Hepatitis-A-Virus enthaltende Stuhlsuspension mit phosphatgepufferter physiologischer Kochsalzlösung (versetzt mit 0,05% Tween® 20) ausgiebig gewaschen. Danach wurden 200 µl Tracer (radioaktiv J$^{125}$-markierte Antikörper gegen Hepatitis-A-Virus) zugegeben und weitere drei bis vier Stunden bei Zimmertemperatur inkubiert. Nach dieser Inkubationszeit wurde der Tracer durch ausgiebiges Waschen mit destilliertem Wasser entfernt und die auf den Kugeln gebundene Radioaktivität in einem Gamma-Zähler gemessen. Als Kontrollen dienten unbeschichtete Kugeln und gleicherart hergestellte Stuhlsuspensionen von Patienten, die keine Hepatitis-A-Viren im Stuhl aufwiesen.

Der gleiche Ansatz erfolgte zum Nachweis der unspezifischen Bindung von Hepatitis-B-Viren bzw. Hepatitis-B-Virusoberflächenantigen. Anstelle der A-Virus-Suspension wurden 200 µl gereinigtes Hepatitis-B-Surface-Antigen (kommerziell erhältlicher gereinigter Impfstoff der Firma LABAZ) oder bekannte Hepatitis-B-Virus haltige Seren eingesetzt.

Beispiel 3
Gewinnung virusbindender Fibronectinbruchstücke

Fibronectin kann enzymatisch mit Subtilisin in mehrere Bruchstücke gespalten werden. Bruchstücke, die Bindungsstellen für Kollagen enthalten, erwiesen sich als ungeeignet für die Bindung von Hepatitisvirusantigenen. Die für die Virusbindung ungeeigneten, d.h. die kollagenbindenden Bruchstücke, lassen sich in einfacher Weise durch Adsorption an Kollagen-Sepharose® abtrennen. Die virusbindenden Bruchstücke werden in bekannter Weise durch chromatographische Methoden nach Molekulargewichten getrennt. Die hierbei gewonnenen Bruchstücke weisen Molekulargewichte zwischen 10 000 und 100 000 auf.

Beispiel 4
Freisetzung von an Fibronectin gebundenen Viren

Die Freisetzung von an Fibronectin gebundenem Hepatitisvirusantigen konnte durch Zugabe von rezeptorzerstörenden Enzymen (Neuraminidase, Chymotrypsin) durch Verdrängungsreaktionen oder durch Erhitzen erreicht werden. Speziell beim gesuchten Non-A-Non-B-Virusantigen (bzw. der mit Non-A-Non-B-Hepatitis assoziierten Substanz) konnte eine besnders effektive Freisetzung durch Zugabe von Neuraminidase (das sogen. receptor destroying enzyme) sowie durch einfaches Erhitzen auf 80°C (10 min) erreicht werden. Für das Hepatitis-A-Virus ist die Freisetzung durch Erhitzen nicht geeignet, da die Antigenität dieses Virus bei diesen relativ hohen Temperaturen zerstört wird; gleichermaßen ist auch beim Hepatitis-A-Virus Chymotrypsin nicht für die Freisetzung geeignet, da dieses Enzym die Antigeneigenschaften des Hepatitis-A-Virus zerstört. Bedingt geeignet für die Freisetzung des Hepatitis-A-Virus-Antigen ist Neuraminidase. Zusammenfassend kann man feststellen, daß die jeweils geeignete Freisetzungsmethode nach spezifischen Viruseigenschaften (Hitzestabilität, Säurestabilität, Größe, Hülle, Stabilität gegenüber Enzymen und Detergentien) ausgewählt werden muß.

Beispiel 5

Reinigung der Virussuspension

Die nach der Freisetzung der Virusantigene vorliegende Suspension enthält im allgemeinen neben den Antigenen Fibronectin oder Fibronectinbruchstücke oder denaturiertes Fibronectin sowie die zur Freisetzung benutzte Substanz (z.B. Neuraminidase). Eine Isolierung des Virusantigens aus dieser Suspension oder eine Abtrennung störender Substanzen ist nur dann erforderlich, wenn solche Substanzen bei Nachfolgereaktionen (z.B. Antigen-Antikörperreaktionen) stören. Eine solche Reinigung erfolgt nach bekannten Methoden. Beispielsweise kann denaturiertes Fibronectin durch Zentrifugieren aus der Virussuspension entfernt werden. Eine weitergehende Reinigung kann durch chromatographische Trennung oder durch Gradientenzentrifugation erfolgen.

Beispiel 6

Maskierung von Virusantigen durch Fibronectin

Soweit festgestellt werden konnte, werden nicht alle gebundenen Virusantigene maskiert. Dies gilt insbesondere für das Hepatitis-B-Oberflächenantigen, das sich im Gegensatz zum (maskierten) Hepatitis-A-Antigen im fibronectinhaltigen Serum nachweisen läßt. Wie das Hepatitis-A-Virusantigen wird auch das Non-A-Non-B-Virusantigen (bzw. das mit Non-A-Non-B-Hepatitis assoziierte Partikel) durch Fibronectin maskiert. Die Maskierung von Hepatitis-A-Viren verhindert nicht, daß sie als Antigene wirksam werden, wie aus der Bildung spezifischer Antikörper gegen Hepatitis-A-Virusantigene folgt.

Beispiel 7

Beweis für die Freisetzung von
Non-A-Non-B-Hepatitis-Virusantigenen
aus Fibronectin

Ein direkter Beweis für die Freisetzung des Non-A-Non-B-Virusantigens aus Fibronectin könnte nur durch eine Infizierung geeigneter Tiere mittels der freigesetzten Substanz bzw. durch die Erzeugung von einer Non-A-Non-B-Hepatitis-Infektion verhindernden Antikörpern erbracht werden. Die Diagnose der Infektion von Tieren durch Non-A-Non-B-Hepatitis-Virus erfolgt durch den morphologischen Nachweis von Leberzellnekrosen und entzündlichen Infiltraten (histologischer Nachweis), den klinisch chemischen Nachweis anhand des Anstieges leberspezifischer Enzyme im Serum sowie den gleichzeitigen Ausschluß anderer Lebererkrankungen, insbesondere den Ausschluß von Hepatitis A und Hepatitis B.

Ein indirekter Beweis für die Freisetzung des Non-A-Non-B-Hepatitis-Virus wurde bei den Untersuchungen in folgender Weise erbracht:

Ausgehend von den Überlegungen, daß Non-A-Non-B-Viren im Blut in freier Form mittels den üblichen Testmethoden mit Radioimmunoassay bisher nicht nachgewiesen werden konnten und daß die Häufigkeit der Infektion bei Gaben von Plasmaderivaten in direkter Korrelation mit dem Fibronectingehalt der verwandten Plasmaderivate bei parenteral übertragener Non-A-Non-B-Hepatitis stand, wurden fibronectinfreie biologische Flüssigkeiten auf die Anwesenheit von Substanzen untersucht, die mit einer Non-A-Non-B-Hepatitis einhergingen. Die Ausscheidung von Viren in verschiedene Körperflüssigkeiten, Sekrete und Exkrete ist bekannt und wird in der Virologie zum Nachweis von Viren eingesetzt (Virusnachweis im Speichel, Stuhl usw.).

Anhand von über 3000 Untersuchungen an Patienten mit sporadischer und parenteral erworbener Non-A-Non-B-Hepatitis sowie an gesunden Kontrollpersonen und Patienten, die an anderen Virushepatitiden und Leberkrankheiten anderer Genese litten, konnte gezeigt werden, daß im Verlaufe der Non-A-Non-B-Hepatitis eine spezielle Substanz im fibronectinfreien Stuhlfiltrat nachzuweisen ist. Der Nachweis dieser Substanz korreliert mit der klinischen Diagnose Non-A-Non-B-Hepatitis. Dieses mit Non-A-Non-B-Hepataitis assoziierte Partikel wurde hinsichtlich seiner chemischen und physikalischen Eigenschaften untersucht. Dazu wurden Stabilität gegen organische Lösungsmittel, Hitzeinaktivierung, Stabilität gegenüber verschiedenen Detergentien, Dichtegradientenzentrifugation im Cäsiumchloridgradienten und Saccharosegradienten durchgeführt. Dieses Partikel läßt sich in gleicher Art wie das Hepatitis-A-Virus durch Fibronectin maskieren und kann auch durch Zugabe von Hepatitis-A-Viren aus seiner Bindung vom Fibronectin verdrängt werden. Entsprechend ist sein Verhalten gegenüber eiweißabbauenden Enzymen und organischen Lösungsmitteln. Entsprechend seinem Sedimentationsverhalten handelt es sich um stabiles partikuläres Material ähnlich einem Virus, das zudem mit einer jahreszeitlichen Häufung in den Wintermonaten und Frühjahrsmonaten ausgeschieden wird.

Methodik: 1 : 10 (G/V) verdünnte Stuhlsuspension von Patienten mit NANB-Hepatitis wurden nach zweimaliger Fällung mit 10% PEG-6000 und Freon-Extraktion mit DNase, RNase und Sarcosyl® (1%ig) inkubiert. Das Sediment wurde auf einem Cäsiumchloridgradienten mit einer Dichte von 1,2 bis 1,4 (g/ml) aufgetragen und 6,24 und 42 h zentrifugiert (30 000 RPM). Die positiven Fraktionen wurden im Saccharose-Gradienten von (5–30%) weiter gereinigt (40 000 RPM) und mittels SDS-PAGE analysiert. Die Stabilität gegenüber Enzymen und Detergenzien wurde mit Chymotrypsin, Trypsin, Elastase, Papain, Pepsin, Pronase, DNase, RNase, Neuraminidase und SDS untersucht. Die Hitzestabilität wurde über einen Bereich von 37 °C bis 100 °C geprüft. Je 50 µg Protein pro ml der Substanz wurden zusammen mit kompletten Freund'schen Adjuvanz zum Immunisierung von Kaninchen eingesetzt. Die Überprüfung der Bindungsaffinität der Substanz erfolgte nach [125]J-Markierung und Inkubation mit normalem menschlichen Serum und anschließender chromatographischer Trennung (Biogel® Ac 34).

Ergebnisse: Ein Hauptpeak der Substanz fand sich konstant bei einer Dichte von 1,25 bis 1,26 (g/ml). In der SDS-PAGE stellten sich zwei Polypeptide mit einem Molekulargewicht bei 40 000 D

dar. Die $^{125}$J-markierte Substanz zeigte eine starke Affinität gegen alle chromatographisch trennbaren Serumfraktionen. Pronase®-, Papain- und Pepsinbehandlung sowie Zusatz von 0,1% SDS zerstörte die Bindungsaffinität des gereinigten Materials, das sich gegenüber den anderen Enzymen sowie gegenüber einer Erhitzung auf 70°C über 45 Min. als stabil erwies. Die mit der Substanz immunisierten Kaninchen bildeten spezifische Antikörper.

Bei prospektiver Untersuchung zur Frage einer posttransfusionären Hepatitis unter Einschluß einer Untersuchung des Stuhls auf das Vorhandensein einer der Hepatitis NANB assoziierten Substanz wurde bei einer Reihenuntersuchung festgestellt, daß 21% der Patienten im Stuhl positiven Befund zeigten, von denen 63% manifest an einer Hepatitis erkrankten. Weitere 25% entwickelten bis zum Stichtag einen Anstieg der Gamma-GT. Nur bei 5,5% ließen sich keine Veränderungen feststellen. Somit ist aus der Sicht eines positiven Tests einer der Hepatitis NANB assoziierten Substanz im Stuhl zu 97,5% der Fälle mit einer vermutlich infektionsbedingten Leberschädigung zu rechnen, falls eine Transfusion vorausgeht. Die Gesamtzahl n der Patienten, bei denen eine genügend lange Dauer nach der Transfusion vorlag, demgemäß eine dem Studienziel entsprechende Diagnose gestellt werden konnte, betrug 38.

Eine weitere Anwendungsmöglichkeit des Verfahrens der Erfindung besteht in einer Abänderung wobei das Fibronectin aus dem Blutplasma vor Freisetzung des Virus bzw. Antigens entfernt und so ein virusfreies Blutplasma erhalten wird. Diese Modifikation ist noch wichtiger bei der Herstellung von Blutplasmafraktionen, z.B. Gammaglobulin oder Faktor 8. Bei der üblichen Herstellung solcher Blutplasmafraktionen findet eine gewisse Anreicherung von Fibronectin statt und die Entfernung des Fibronectins entfernt gleichzeitig alle Viren bzw. bindungsfähige Antigene, so daß virenfreie Plasmafraktionen erhalten werden.

Der Ausdruck «Fibronectin», wie er hier verwendet wird, bezieht sich insbesondere auf menschliches Fibronectin und auf das Fibronectin von Primaten allgemein. Da Fibronectin artspezifisch ist, können Unterschiede bestimmter aktiver Gruppen der Fibronectinstruktur auftreten die die Bindungsfähigkeit für Viren, insbesondere Non-A-Non-B-Virus bzw. die entsprechenden Antigene, nicht auftreten lassen. Im Lichte der obigen Beschreibung kann dies jedoch von Fall zu Fall bestimmt werden, wenn mit einem anderen Fibronectin als dem von Primaten gearbeitet werden soll.

**Patentansprüche**

1. Verfahren zur Gewinnung von Viren bzw. Virusantigen aus biologischem Material, insbesondere Körperflüssigkeiten, dadurch gekennzeichnet, daß man als Ausgangsmaterial eine fibronectinhaltige Körperflüssigkeit oder ein mit Fibronectin versetztes biologisches Material verwendet und das Virus bzw. das Virusantigen durch an sich bekannte Zerstörung des Bindungsmechanismus, insbesondere durch spezielle Enzyme und/oder Detergenzien oder durch Verdrängungsreaktionen oder Erhitzen freisetzt und gegebenenfalls aus der erhaltenen Suspension die Viren bzw. das Virenantigen isoliert und gewünschtenfalls in an sich bekannter Weise reinigt.

2. Verfahren nach Anspruch 1, dadurch gekennzeichnet, daß man statt Fibronectin Fibronectinbruchstücke, die zur Bindung von bestimmten Viren bzw. Virenantigen befähigt sind, einsetzt.

3. Verfahren nach Anspruch 1 oder 2, dadurch gekennzeichnet, daß man Non A-Non B-Hepatitisvirus bzw. -virusantigen aus Fibronectin oder dessen Bruchstücken gewinnt.

4. Verfahren nach einem der vorhergehenden Ansprüche, dadurch gekennzeichnet, daß man als Körperflüssigkeit Blut oder als biologisches Material ein Stuhlhomogenisat verwendet.

5. Verfahren nach einem der vorhergehenden Ansprüche, dadurch gekennzeichnet, daß man die Freisetzung des Virus bzw. des Virusantigens mit Neuraminidase oder Chymotrypsin durchführt.

6. Verfahren nach einem der vorhergehenden Ansprüche, dadurch gekennzeichnet, daß man die Freisetzung des Virus bzw. Virusantigens durch Erhitzen durchführt.

7. Anwendung des Verfahrens nach einem der Ansprüche 1 bis 6, insbesondere Anspruch 4 für Screening-Tests, insbesondere zur Früherkennung von Vireninfektionen durch Stuhluntersuchung, insbesondere für Posttransfusions-Hepatitis.

8. Anwendung des Verfahrens nach Anspruch 1 zur Gewinnung von virus- bzw. antigenfreien Blutplasmafraktionen durch Entfernen des Fibronectins mit dem daran gebundenen Virus oder Antigen aus dem Plasma vor der Freisetzung des Virus bzw. Virusantigens.

9. Diagnosekit zur Diagnose von durch Fibronectin oder dessen Bruchstücken maskierten Viren bzw. Virenantigenen, enthaltend neben markierten Antigenen sowie markierten und nicht markierten spezifischen Antikörpern gegen diese Antigene zusätzlich Reagenzien, welche die Freisetzung der gebundenen Viren bzw. Virenantigene aus Fibronectin oder dessen Bruchstücken ermöglichen.

10. Diagnosekit nach Anspruch 9, dadurch gekennzeichnet, daß zur Freisetzung von gebundenem Virus bzw. Antigen Neuraminidase oder Chymotrypsin und gegebenenfalls Kollagen-Sepharosefilter enthalten sind.

**Claims**

1. Process for obtaining viruses or viral antigen from biological material, especially body fluids, characterized in that the starting material used is a fibronectin-containing body fluid or a biological material to which fibronectin has been added, and the virus or the viral antigen is released by destruction, which is known per se, of the mechanism of binding, especially by specific enzymes and/or detergents or by displacement reactions or heating, and, where appropriate, the viruses or the viral antigen is isolated from the resulting suspension and, if desired, further purified in a manner known per se.

2. Process according to Claim 1, characterized in that in place of fibronectin fibronectin fragments which are able to bind particular viruses or viral antigen are used.

3. Process according to Claim 1 or 2, characterized in that non-A, non-B hepatitis virus or viral antigen thereof is obtained from fibronectin or fragments thereof.

4. Process according to one of the preceding claims, characterized in that blood is used as body fluid or a stool homogenate is used as biological material.

5. Process according to one of the preceding claims, characterized in that the release of the virus or of the viral antigen is carried out with neuraminidase or chymotrypsin.

6. Process according to one of the preceding claims, characterized in that the release of the virus or viral antigen is carried out by heating.

7. Use of the process according to one of Claims 1 to 6, especially Claim 4 for screening tests, especially for the early diagnosis of viral infections by examination of stool, especially for post-transfusion hepatitis.

8. Use of the process according to Claim 1 for obtaining blood plasma fractions which are free of virus or antigen by removing the fibronectin with the antigen or virus bound thereto from the plasma before the release of the virus or viral antigen.

9. Diagnosis kit for the diagnosis of viruses or viral antigens masked by fibronectin or the fragments thereof, containing besides labelled antigens together with labelled and unlabelled specific antibodies against these antigens additionally reagents which make possible the release of the bound viruses or viral antigens from fibronectin or the fragments thereof.

10. Diagnosis kit according to Claim 9, characterized in that neuraminidase or chymotrypsin and, where appropriate, collagen-Sepharose filters are contained for the release of bound virus or antigen.

**Revendications**

1. Procédé d'obtention de virus ou d'antigène de virus à partir d'une matière biologique, notamment liquides du corps, caractérisé en ce qu'on utilise comme matière première un liquide corporel contenant de la fibronectine ou une matière biologique mélangée à de la fibronectine et en ce qu'on libère le virus ou l'antigène de virus, par destruction connue en soi du mécanisme de liaison notamment par enzymes spéciales et/ou détergents ou par réaction de déplacement ou par chauffage et en ce que, le cas échéant, on isole de la suspension ainsi obtenue, les virus ou l'antigène de virus qui, si souhaité, sont purifiés d'une manière connue en soi.

2. Procédé suivant la revendication 1, caractérisé en ce qu'on met en œuvre, à la place de la fibronectine, des fragments de fibronectine qui sont aptes à lier des virus ou un antigène de virus déterminés.

3. Procédé suivant la revendication 1 ou 2, caractérisé en ce qu'à partir de la fibronectine ou de ses fragments on obtient des virus ou des antigènes de virus de l'hépatite non-A non-B.

4. Procédé suivant l'une des revendications précédentes, caractérisé en ce qu'on utilise comme liquide corporel, du sang, ou comme matière biologique, un homogénisat des selles.

5. Procédé suivant l'une des revendications précédentes, caractérisé en ce qu'on procède à la libération du virus ou de l'antigène du virus avec de la neuraminidase ou de la chymotrypsine.

6. Procédé suivant l'une des revendications précédentes, caractérisé en ce qu'on procède à la libération du virus ou de l'antigène du virus par chauffage.

7. Utilisation du procédé suivant l'une des revendications 1 à 6, en particulier la revendication 4 pour des tests de sélection (screening), en particulier pour la détermination précoce d'infections virales par analyse de selles, notamment pour des hépatites post-transfusionnelles.

8. Utilisation du procédé suivant la revendication 1 pour l'obtention de fractions de plasma sanguin exemptes de virus ou d'antigène de virus par élimination du plasma de la fibronectine liant virus ou antigène avant la libération du virus ou de l'antigène.

9. Kit de diagnostic pour diagnostiquer des virus ou des antigènes de virus camouflés par la fibronectine ou ses fragments, comportant des antigènes marqués et des anticorps spécifiques marqués et non marqués, dirigés contre ces antigènes, ainsi que des réactifs supplémentaires aptes à assurer la libération des virus ou des antigènes de virus liés à la fibronectine ou à ses fragments.

10. Kit de diagnostic selon la revendication 9, caractérisé en ce qu'il comporte de la neuraminidase ou de la chymotrypsine et le cas échéant un filtre sepharose-collagène pour la libération du virus ou de l'antigène du virus lié.